# FASCICULE DE BREVET EUROPEEN

(11) **EP 4 326 190 B1**
(45) Date de publication et mention de la délivrance du brevet: **12.02.2025**
(21) Numéro de dépôt: 22727264.8
(22) Date de dépôt: 20.04.2022
(51) Int. Cl.: A61F 2/04

(54) **PROTHÈSE D'OESOPHAGE IMPLANTABLE**
IMPLANTIERBARE ÖSOPHAGUSPROTHESE
IMPLANTABLE OESOPHAGEAL PROSTHESIS

(30) Priorité: 20.04.2021 BE 202105309
(43) Date de publication de la demande: 28.02.2024
(73) Titulaire: Voigt, Kerstin-Evelyne, 66700 Argelès sur mer (FR)
(72) Inventeur: Voigt, Kerstin-Evelyne, 66700 Argelès sur mer (FR)
(74) Mandataire: Quintelier, Claude
(86) Numéro de dépôt international: PCT/EP2022/060355
(87) Numéro de publication internationale: WO 2022/223578

(56) Documents cités:
- WO-A1-2019/241414
- FR-A1- 2 621 813
- US-A1- 2018 125 633

## Description

La présente invention concerne une prothèse d'œsophage implantable comprenant un tube alimentaire, lequel tube alimentaire est au moins partiellement entouré d'un logement dans lequel un organe pressuriseur est appliqué, lequel organe pressuriseur comprend un élément d'application de pression agencé pour générer une pression péristaltique contre une paroi externe du tube alimentaire.

Une telle prothèse d'œsophage implantable est connue de WO2019/241414 et est utilisée pour remplacer un œsophage naturel dans le corps d'une personne. La prothèse d'œsophage implantable connue comprend un tube, réalisé par exemple en silicone, pour des applications biomédicales. La prothèse connue comprend également une pluralité de logements dans lesquels est chaque fois logé un organe pressuriseur comprenant un élément d'application de pression agencé pour générer une pression péristaltique contre une paroi externe du tube alimentaire et ainsi contribuer au déplacement d'aliment dans le tube alimentaire.

Un inconvénient de la prothèse d'œsophage implantable connue est que due à l'usage d'une pluralité de logements il n'est pas possible d'appliquer une pression continue sur la longueur de l'œsophage implanté. En effet la pression est appliqué à l'endroit où le logement est situé mais pas sur la partie entre deux logements successifs. Cela pourrait conduire à une situation dans laquelle les aliments introduits pourraient se bloquer dans la prothèse œsophagienne implantée entre deux logements successifs et ainsi nuire à la personne dans laquelle la prothèse est implantée. La fiabilité de la prothèse d'œsophage implantable connue n'est donc pas suffisante pour une application sur un corps humain.

La présente invention a pour objet de réaliser une prothèse d'œsophage implantable qui est plus fiable.

A cet effet, la prothèse d'œsophage implantable selon la présente invention est caractérisée en ce que l'organe pressuriseur est appliqué de telle manière à pouvoir être déplacé le long du tube alimentaire sur une distance prédéterminée à partir d'une position initiale, lequel élément d'application de pression est agencé pour générer la pression péristaltique de façon continue pendant son déplacement le long du tube alimentaire dans une première direction s'étendant vers l'estomac d'un corps dans lequel la prothèse doit être implantée, ledit organe pressuriseur comprenant un organe de rappel agencé pour appliquer une force de rappel s'étendant dans une seconde direction, opposé à la première direction, pour ramener l'élément d'application de pression vers sa position initiale. En générant une pression péristaltique continue contre la paroi externe du tube alimentaire pendant son mouvement le long du tube alimentaire dans une première direction s'étendant vers un estomac d'un corps humain dans lequel la prothèse doit être implantée, on veille à ce qu'une pression continue soit appliquée contre la nourriture présente dans le tube alimentaire et à transporter cette nourriture vers l'estomac. Ceci permet de transporter les aliments à travers la prothèse d'œsophage de manière fiable, réduisant ainsi considérablement le risque que les aliments se bloquent dans la prothèse d'œsophage.

On notera que la demande de brevet US 2018/0125633 décrit un dispositif qui peut être implanté dans l'œsophage naturel d'un être vivant et qui peut se déplacer dans cet œsophage et répondre à des forces produites par de mouvements péristaltiques de l'œsophage naturel. Toutefois le dispositif décrit dans US 2018/0125633 ne peut être combiné avec la prothèse décrite dans WO2019/241414. En effet le dispositif selon US 2018/0125633 est placé à l'intérieur d'un œsophage naturel alors que dans WO2019/241414 l'organe pressuriseur est placé à l'extérieur d'une prothèse qui remplace l'œsophage naturel. Une combinaison de l'enseignement de ces deux demandes de brevets est donc techniquement parlent impossible.

Un premier mode de réalisation préférentiel d'une prothèse d'œsophage implantable selon l'invention est caractérisé en ce que l'élément d'application de pression est agencé pour générer la pression péristaltique continue en utilisant un fluide hydraulique ou un gaz sous pression. L'utilisation d'un fluide hydraulique ou d'un gaz sous pression offre une solution appropriée pour appliquer la pression péristaltique continue contre une paroi externe du tube implanté dans un corps humain.

De préférence, le premier mode de réalisation préférentiel d'une prothèse d'œsophage implantable selon l'invention est caractérisé en ce que l'élément d'application de pression comprend une entrée de fluide et une sortie de fluide, lequel élément d'application de pression comporte une paroi interne configurée pour envelopper au moins partiellement le tube alimentaire et pourvue d'au moins une première série de portes de sortie et au moins une deuxième série de portes d'entrée, les portes d'entrée étant appliquées, considérées dans ladite première direction, sous les portes de sortie, les portes de sortie, respectivement d'entrée, étant reliées à ladite entrée de fluide, respectivement à ladite sortie de fluide, l'entrée de fluide étant reliée à une sortie d'une pompe agencée pour fournir le fluide hydraulique à une pression d'au moins 2 bars. Les portes de sortie permettent une sortie directe du fluide créant ainsi la pression péristaltique continue contre la paroi externe du tube. Les portes d'entrée permettent à leur tour de récupérer le fluide évacué par les portes de sortie et ainsi d'obtenir un circuit de fluide fermé.

De préférence, le premier mode de réalisation préférentiel d'une prothèse d'œsophage implantable selon l'invention est en outre caractérisé en ce que l'organe de rappel est formé par un ressort logé à l'intérieur dudit logement. Ceci permet un retour rapide et fiable du corps cylindrique dans la position initiale.

Un deuxième mode de réalisation préférentiel d'une prothèse d'œsophage implantable selon l'invention est caractérisé en ce que l'élément d'application de pression comprend un ensemble d'organes de compression agencés pour exercer un mouvement d'aller-retour par rapport au tube alimentaire. Les organes de compression offrent une solution mécanique fiable.

De préférence le deuxième mode de réalisation préférentiel d'une prothèse d'œsophage implantable selon l'invention est caractérisé en ce que l'élément d'application de pression comprend un poussoir et un compresseur montés sur un organe d'entraînement agencé pour générer ledit déplacement le long du tube, ledit compresseur étant monté en aval dudit poussoir, lesdits organes de compression faisant partie dudit compresseur, ledit poussoir étant agencé pour imposer un mouvement auxdits organes de compression afin de permettre ledit mouvement d'aller-retour. L'utilisation d'un poussoir et d'un compresseur permet de gérer facilement en fonction des séquences temporelles suivantes le mouvement d'aller-retour et le mouvement le long du tube.

De préférence, le deuxième mode de réalisation préférentiel d'une prothèse d'œsophage implantable selon l'invention est caractérisé en ce que les éléments de compression sont chacun formés par un rouleau appliqué de manière rotative sur un axe. L'utilisation de rouleaux appliqués en rotation permet de réduire le frottement entre les éléments de compression et le tube, réduisant ainsi la consommation d'énergie et augmentant la durée de vie de la prothèse.

L'invention sera maintenant décrite plus en détail en se référant aux dessins annexés. Dans les dessins :
La figure 1 montre un premier mode de réalisation préférentiel d'une prothèse d'œsophage implantable selon l'invention ;
La figure 2 montre un mode de réalisation préférentiel de l'élément d'application de pression ;
La figure 3 montre une coupe transversale dans la direction longitudinale à travers l'élément d'application de pression ;
La figure 4 illustre le fonctionnement du premier mode de réalisation préférentiel d'une prothèse d'œsophage implantable selon l'invention ;
La figure 5 illustre la commande de la pompe ;
La figure 6 montre un exemple du deuxième signal de déclenchement ;
La figure 7 montre un deuxième mode de réalisation de la prothèse d'œsophage implantable selon l'invention ;
La figure 8 illustre l'élément d'application de pression du deuxième mode de réalisation ;
La figure 9 illustre le compresseur du deuxième mode de réalisation ;
La figure 10 montre différentes formes des fentes ;
La figure 11 montre une coupe transversale à travers un exemple de la soupape de commande de passage d'aliments 7 à placer dans le tube d'aliments ; et
La figure 12 montre une forme de réalisation particulière de la pompe.

Dans les dessins, une même référence a été attribué à un élément identique ou analogue.

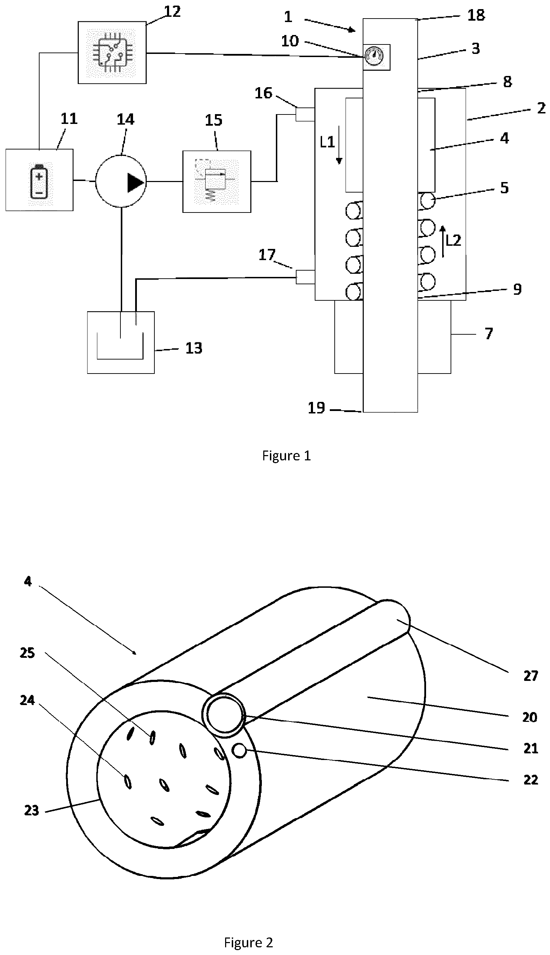

La figure 1 montre un premier mode de réalisation préférentiel d'une prothèse d'œsophage implantable 1 selon l'invention. La prothèse d'œsophage implantable comprend un tube alimentaire 3 agencé pour remplacer un œsophage naturel dans le corps humain d'une personne. Le tube alimentaire est au moins partiellement entouré par un logement 2 dans lequel un organe pressuriseur 4,5 est appliqué de manière à pouvoir être déplacé le long du tube sur une distance prédéterminée à partir d'une position initiale 8, où l'organe pressuriseur est situé dans la partie supérieure du logement, comme indiqué sur la figure 1.

L'organe pressuriseur comprend un élément d'application de pression 4 agencé pour générer une pression péristaltique continue contre une paroi externe du tube alimentaire 3 lors de son déplacement le long du tube dans une première direction I1 s'étendant vers un estomac du corps humain dans lequel la prothèse est implantée. L'organe pressuriseur comprend également un organe de rappel 5, par exemple formé par un ressort, agencé pour appliquer une force de rappel s'étendant dans une deuxième direction I2, opposée à la première direction I1, pour ramener l'élément d'application de pression dans la position initiale 8. Dans le mode de réalisation représenté un ressort de compression est utilisé. Alternativement, un ressort de tension pourrait être utilisé qui serait alors appliqué sur le côté supérieur de l'élément d'application de pression.

La prothèse d'œsophage implantable 1 comprend de préférence également une soupape de contrôle de passage alimentaire 7 placée au niveau de la partie la plus basse du logement correspondant à une position finale 9 de l'organe pressuriseur. La soupape de contrôle du passage des aliments sert d'une part à permettre aux aliments transportés d'accéder à l'estomac. D'autre part, la soupape de contrôle du passage des aliments permet au contenu de l'estomac de refluer via le tube alimentaire en cas de vomissements. La soupape de contrôle de passage des aliments sert également à faire une séparation entre la zone de la bouche de la personne et son estomac, de sorte que les odeurs de l'estomac n'atteignent pas la bouche. La soupape s'ouvrira lorsque l'aliment aura atteint la position finale 9 dans le tube d'aliment 3. La pression d'ouverture est de préférence réglée à 25 mbar telle qu'elle est réalisée dans le corps humain. Dès que l'aliment a passé la soupape, la soupape se referme, par exemple au moyen d'une force de rappel appliquée par un autre ressort.

La soupape fermera également le retour de l'estomac vers la bouche afin de ne pas permettre au liquide provenant de l'estomac de retourner dans la bouche au cas où le patient aurait à vomir. Cela conduirait à une pression très élevée à l'intérieur de l'estomac et potentiellement à de graves dommages causés par une surpression. Par conséquent, la soupape doit permettre aux aliments de prendre la direction opposée, cette fois de l'estomac vers la bouche. Dans ce cas, la pression d'ouverture doit être réglée à une valeur élevée comprise par exemple entre 150 et 200 mbar. En effet, car en cas de gaz dû à des boissons gazeuses ou des vomissements, des pressions élevées surviennent et il doit y avoir une séparation solide entre le mode normal et le mode vomissement. Une description plus détaillée d'une telle soupape anti-reflux et anti-vomissement sera fournie ci-dessous.

La prothèse d'œsophage implantable 1 comprend de préférence également un capteur 10 de détection de passage alimentaire monté en amont de l'élément 4 d'application de pression sur le tube alimentaire 3 et connecté à une unité de contrôle 12 et une unité d'alimentation 11, par exemple une batterie. L'unité de contrôle est également connectée à une pompe 14 qui est alimentée par l'unité d'alimentation 11. La pompe a une ligne d'entrée reliée à un réservoir de fluide 13 et une ligne de sortie reliée via une soupape de limitation de pression 15 à une entrée de fluide 16 du logement 2. Le réservoir de fluide est agencé pour contenir un fluide hydraulique tel que par exemple de l'eau, ou un gaz sous pression. La pompe est agencée pour fournir le fluide hydraulique à une surpression par rapport à la pression atmosphérique de 0,5 à 2 bars. Le logement comprend en outre une sortie de fluide 17 reliée au réservoir de fluide.

Le capteur 10 de détection de passage d'aliments est de préférence un capteur de pression agencé pour mesurer une pression exercée sur le tube alimentaire par l'aliment présent à l'intérieur. Le capteur 10 de détection de passage alimentaire peut également être un détecteur utilisant un faisceau lumineux, un détecteur ultrason ou un manomètre. Le but du capteur de détection de passage d'aliments est de détecter que des aliments sont entrés dans le tube alimentaire. Lorsqu'un détecteur de faisceau lumineux est utilisé, il émettra alors un faisceau lumineux et détectera l'interruption du faisceau lumineux par les aliments. Le détecteur ultrason émettrait un faisceau ultrason et les changements de réflexion de ce faisceau provoqués par la nourriture sont détectés. Dans un souci de clarté, la description sera toutefois limitée à un capteur de pression.

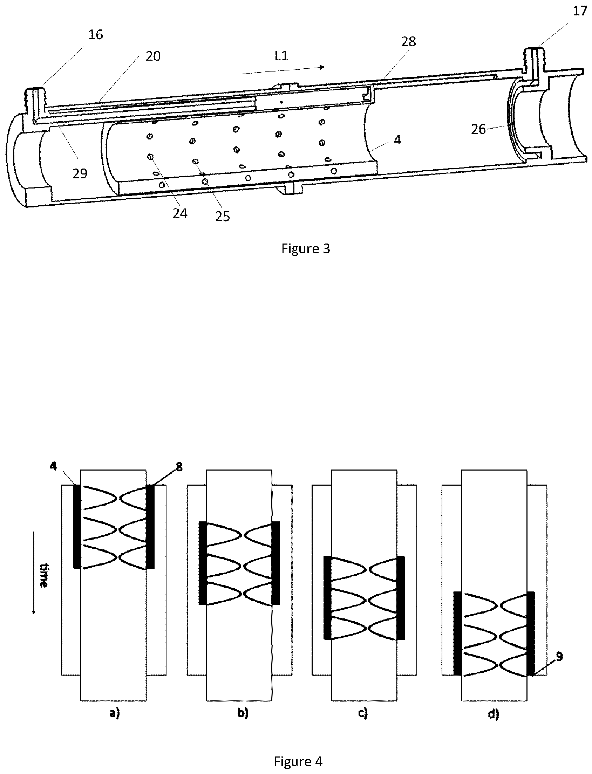

Les figures 2 et 3 montrent un mode de réalisation préférentiel de l'élément 4 d'application de pression. Une découpe partielle est présentée à la figure 2 de manière à montrer une partie de la structure interne de l'élément d'application de pression. La figure 3 montre une coupe transversale dans le sens longitudinal, direction à travers l'élément d'application de pression. L'élément d'application de pression comprend un corps 20, de préférence de forme cylindrique, ayant une entrée de fluide 21 et une sortie de fluide 22. L'utilisation d'un corps de forme cylindrique permet de s'accoupler de manière adéquate avec le tube cylindrique. L'élément d'application de pression comporte également une paroi intérieure 23 pourvue d'au moins une première série de portes de sortie 24 et d'au moins une deuxième série de portes d'entrée. 25. Les portes d'entrée étant appliquées, considérées dans ladite première direction I1, sous les portes de sortie. Les portes de sortie 24, respectivement d'entrée 25, étant reliées à l'entrée de fluide 21, respectivement à la sortie de fluide 22. L'entrée de fluide 21 coopère avec l'entrée de fluide 16 et la sortie de fluide 22 coopère avec la sortie de fluide 17. La première série des portes de sortie 24 comprennent au moins deux portes de sortie et la deuxième série de portes d'entrée comprend au moins une porte d'entrée.

Comme le montre la figure 3, un tube de pression 29 est connecté à l'entrée de fluide 16 et s'étend parallèlement à la paroi interne du corps 20. Le tube de pression 29 s'étend dans une chambre interne 28 de l'élément d'application de pression 4, laquelle chambre interne part de l'entrée de fluide. Cette chambre interne 28 a une paroi de fond reliée à la première série de portes de sortie 24. Les portes d'entrée sont reliées à une autre chambre (non représentée) qui est reliée à la sortie de fluide 22.

L'élément 4 d'application de pression comprend de préférence une barre de guidage 27 appliquée sur la paroi externe du corps 20 et engagée dans un canal de guidage (non représenté) appliqué sur la paroi interne du logement 2. La barre de guidage 27 et le canal de guidage évitent que le corps de forme cylindrique ne tourne à l'intérieur du logement pendant son mouvement à l'intérieur du logement, endommageant ainsi le tube de pression 29.

La figure 4 illustre le fonctionnement du premier mode de réalisation préférentiel d'une prothèse d'œsophage implantable 1 selon l'invention. Lorsque les aliments atteignent une entrée du tube 3 alimentaire, le capteur 10 de pression détecte la pression appliquée par les aliments sur le tube alimentaire. Le capteur 10 de pression génère alors un premier signal de déclenchement dont la valeur dépend de la pression appliquée par l'aliment dans le tube alimentaire. Le premier signal de déclenchement est fourni à l'unité de contrôle 12 qui vérifie la valeur fournie par le premier signal de déclenchement. Si cette dernière valeur dépasse une valeur seuil prédéterminée, l'unité de contrôle génère un second signal de déclenchement qui est fourni à la pompe 14. Sous la commande du second signal de déclenchement, la pompe est activée et commence à pomper du fluide hors du réservoir de fluide 13. Le fluide pompé est amené sous pression vers l'entrée de fluide 16 et atteindra ainsi le tube de pression 29.

Puisque le tube de pression 29 s'étend dans la chambre intérieure 28, cette dernière va se remplir de fluide sous pression qui atteindra alors la première série de portes de sortie 24. Le fluide sous pression va s'évacuer par la première série de portes de sortie 24 et sera éjecté sous pression contre la paroi externe du tube alimentaire appliquant ainsi une pression péristaltique continue contre la paroi externe du tube 3, comme illustré sur la figure 4a. Dans le mode de réalisation représenté à la figure 4a, la première série de portes de sortie comprend trois rangées de portes successives, ce qui explique pourquoi trois oscillations de pression de forme d'onde consécutives sont représentées. On comprendra que le nombre de trois oscillations n'est donné qu'à titre d'exemple et qu'au moins une oscillation suffirait à créer une pression sur la paroi externe du tube alimentaire.

En éjectant le fluide contre la paroi externe du tube alimentaire au moyen de la première série de portes de sortie, le tube alimentaire se contracte localement. Une fois la compression ciblée du tube alimentaire atteinte, la pression du fluide va provoquer le déplacement de l'élément d'application de pression le long de l'axe I1 à partir de la position initiale 8 vers la position finale 9, comme illustré successivement sur les figures 4b, c et d. La pression appliquée contre le tube alimentaire et le mouvement de l'élément d'application de pression amèneront les aliments, présents dans le tube, à se déplacer vers l'estomac. Ce mouvement agit également contre l'organe de rappel 5.

Tant que l'élément d'application de pression se déplace le long de l'axe I1, l'effet de compression est toujours actif, c'est-à-dire que l'endroit où la compression a lieu se déplace avec l'élément d'application de pression. Ce mouvement de contraction produit l'onde de compression de pression péristaltique continue requise appliquée sur le tube alimentaire. Une fois que l'élément d'application de pression a atteint la position finale 9 située au fond du logement, la pompe 14 cessera de débiter. L'effet est une diminution rapide de la pression appliquée et donc une baisse de la pression appliquée contre le tube alimentaire. L'effet est que la compression du tube alimentaire s'arrête et que l'organe de rappel repoussera alors l'élément d'application de pression vers sa position initiale 8.

Entre deux rangées de la première série de portes de sortie 24, il y a une rangée de la deuxième série de portes d'entrée 25 à travers lesquelles le fluide éjecté peut être collecté après avoir appliqué la pression contre le tube alimentaire de manière à éviter une « compression par bloc » du tube alimentaire. Le nombre de séries de portes d'entrée est de préférence inférieur d'un au nombre de série de portes de sortie. Au niveau de l'espace à la position initiale 8 et à l'espace à la position finale 9, l'élément d'application de pression doit être connecté au chemin de retour 26 de sorte qu'au-dessus et au-dessous, la contre-pression s'applique toujours. Le fluide collecté par les portes d'entrée 25 est temporairement stocké dans l'autre chambre pendant le mouvement vers le bas de l'élément d'application de pression et est acheminé vers le trajet de retour 26 lorsque l'organe d'application de pression a atteint sa position finale. Le trajet de retour est connecté à la première sortie de fluide de manière à permettre un reflux du fluide vers le réservoir de fluide 13. De cette manière, le fluide est maintenu dans un circuit fermé et la prothèse peut fonctionner comme un dispositif autonome à l'intérieur de la personne dans laquelle elle est implantée. De façon optionnelle une pompe aspirante peut être utilisée pour ramener le fluide vers le réservoir de fluide. Une telle pompe aspirante est actionnée lorsque l'élément d'application de pression a atteint sa position la plus basse dans le logement.

Dans le mode de réalisation représenté à la figure 1, le réservoir de fluide est à l'extérieur du logement, mais alternativement, le réservoir de fluide et / ou la pompe pourraient également être incorporés dans le logement. Il en est de même pour l'unité de contrôle 12 et l'unité d'alimentation 11. L'unité d'alimentation peut également être réalisée à l'intérieur d'un blister et configurée comme une unité enfichable qui offrirait la possibilité de remplacer facilement l'unité d'alimentation lorsqu'elle est vide. Lorsqu'une unité enfichable est utilisée, il faut veiller à ce que la connexion soit étanche aux fluides et à ce que l'unité soit stérilisée. Il est en outre possible de recharger la batterie par induction.

La dimension du tube alimentaire 3 sera choisie en fonction de la morphologie du patient. De préférence, le tube alimentaire est fabriqué en utilisant un procédé d'impression 3D, en particulier un procédé stéréolithographie, dans lequel un matériau à base de résine est utilisé. Le matériel utilisé doit bien entendu être biocompatible et peut par exemple utiliser des cellules biologiques. Le tube alimentaire a quatre points de connexion à considérer, c'est-à-dire deux joints 18 et 19 vers les extrémités restantes du tube alimentaire de la personne et deux connexions avec le logement de l'œsophage artificiel 2. Le tube alimentaire est de préférence réalisé à partir d'une pièce formée permettant les extrémités réalisées à partir d'un tube en silicone très souple qui permettra d'être scellé aux extrémités restantes du tube alimentaire du patient. A ces extrémités, il n'y a pas d'exigences particulières concernant la rondeur du tube. Cependant, au niveau des points de connexion au corps principal, il y a de préférence des incrustations mécaniques intégrées dans le tube alimentaire qui appliqueront une pression mécanique au niveau de la connexion entre le tube et le corps principal dans le but de sceller le tube alimentaire vers l'extérieur. Ces incrustations peuvent être en métal ou en tout autre matériau adapté pour appliquer une contrainte au point de connexion.

L'intérieur du tube alimentaire est de préférence formé avec une forte exigence au niveau de la circularité, en particulier pour le premier mode de réalisation utilisant un fluide sous pression ou un gaz. **Il** faut veiller à ce que le tube reprenne une forme ronde, de préférence en moins d'une seconde, après chaque compression afin d'éviter tout frottement mécanique avec le pressuriseur en mouvement. Dans le même temps, l'épaisseur de paroi du tube ainsi que sa dureté doivent être bien choisies, suffisamment rigides pour retrouver sa forme ronde après compression mais permettent toujours la compression avec le moins d'effort. Des tests ont montré qu'une combinaison d'une épaisseur de paroi de 2-3 mm et d'une dureté de 50 Shore A peut répondre aux besoins. L'étanchéité aux fluides est également une exigence de la prothèse. C'est pourquoi, en plus des étanchéités mécaniques, un deuxième type de joint est agencé, par exemple avec de la colle. Une souplesse globale de la prothèse est en outre avantageuse pour pouvoir l'ajuster à la morphologie corporelle du patient et pouvoir suivre les mouvements imposés à la prothèse implantée.

De préférence, le tube alimentaire est réalisé en utilisant un matériau appelé « Elastic 50A » et commercialisé par Fa. Formlabs (https://support.formlabs.com/s/article/Using-Elastic- Resin ? Language = en_US). La résine Elastic 50A est un matériau élastomère conçu pour les applications nécessitant un allongement élevé et un retour d'énergie élevé. Ce matériau est particulièrement adapté aux objets qui doivent être pliés, étirés, comprimés et résister à des cycles répétés sans se déchirer. Le matériau est également transparent, ce qui le rend bien adapté aux modèles médicaux pour la simulation ou l'éducation. En comparaison avec d'autres matériaux, l'élasticité a la capacité de reprendre sa forme normale après avoir été étirée ou comprimée. La résine Elastic 50A est conçue pour « rebondir » et reprendre rapidement sa forme d'origine. Le duromètre étant la dureté d'un matériau, la résine Elastic 50A a un duromètre inférieur à celui des autres résines Formlabs, ce qui la rend appropriée pour le prototypage de pièces normalement produites avec du silicone. La résine Elastic 50A a également une résistance à la déchirure plus élevée que les matériaux comparables, ce qui la rend capable de cycles d'utilisation répétés.
Les paramètres physiques d'Elastic 50A sont :
Duromètre / dureté Shore : 50A
Allongement à la rupture (%) : 160
Résistance à la traction (MPa) : 3,2
Résistance au déchirement (kN / m) : 19,1
Couleur claire

De plus, l'aspect de l'avoir sous forme de matériau translucide est également important, car il permet d'utiliser des colles UV actives pour les scellements. Le matériau se présente sous la forme d'une résine fluide qui est ensuite imprimée dans le processus dit SLA (stéréolithographie).

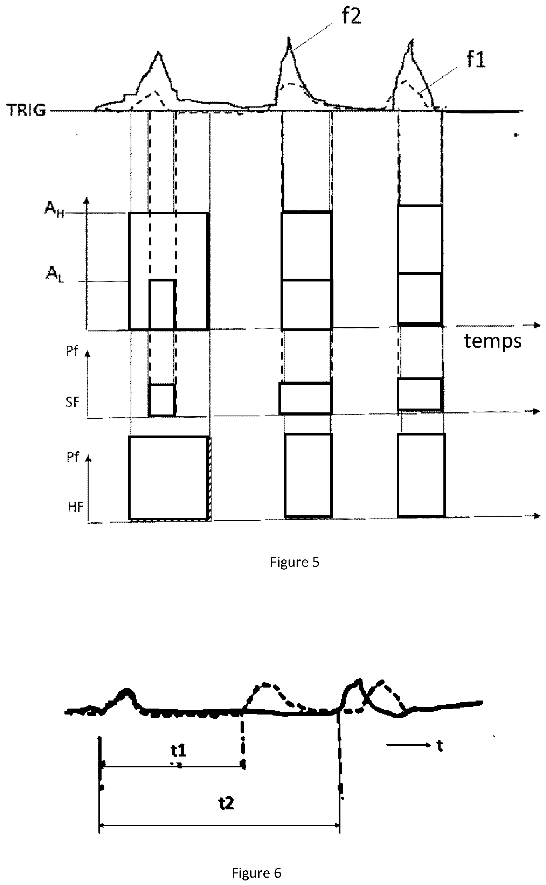

La pression à appliquer sur le tube alimentaire dépend de la nature de l'aliment. Si l'aliment est dur, il doit y avoir une compression élevée du tube alimentaire et par conséquent l'unité de contrôle 12 demandera un débit de distribution élevé à la pompe. Ceci se traduit par la vitesse de la pompe qui équivaut au débit de la pompe et est illustré sur la figure 5. Sur la figure 5, la courbe f1, respectivement f2, montre la valeur du premier signal de déclenchement généré par le capteur de pression 10 en cas de détection de nourriture douce, respectivement dure. L'amplitude du signal de la courbe f1 est inférieure à celle de la courbe f2, car une pression plus faible a été détectée pour les aliments mous. De plus, le temps pendant lequel le capteur de pression détectera la pression appliquée par l'aliment est pris en compte lors de la génération du premier signal de déclenchement. Le premier signal de déclenchement sera ensuite utilisé par l'unité de contrôle pour déterminer l'amplitude et la période du second signal de déclenchement fourni à la pompe. Comme illustré sur la figure 5, l'amplitude et la période du second signal de déclenchement AL, généré lorsqu'un aliment mou (SF) est détecté, est inférieure et plus courte que celle de AH lorsqu'un aliment plus dur (HF) est détecté.

L'unité de contrôle est également agencée pour analyser la fréquence de déglutition, car les patients mangent à des vitesses différentes, ainsi par exemple certains patients mangeront lentement, tandis que d'autres mangeront vite. Cette fréquence de déglutition est déterminée en considérant deux événements qui se suivent lorsque l'amplitude du signal de capteur f1 et f2 dépasse le niveau de déclenchement. Ce temps caractéristique est représenté par t1 et t2 à la figure 6. La pompe sera entraînée avec des ensembles de phases de pompe active et passive. Si le patient mange lentement, le temps entre deux d'entre eux sera plus long. Plus le patient mange vite, plus l'ensemble de ces événements se rapproche.

Le médecin du patient pourrait être intéressé à analyser le fonctionnement de la prothèse d'œsophage implantée chez le patient ainsi que le comportement de déglutition du patient pour un traitement adapté. Par conséquent, l'œsophage implantable sera de préférence équipé de moyens permettant une transmission sans fil des données requises. Dans ce dernier cas, l'unité de contrôle sera équipée d'une mémoire pour stocker les données mesurées et/ou produits et munie d'une unité de transmission WIFI, Bluetooth ou équivalente afin de lire les données stockées.

Il pourrait également être intéressant de régler ou d'adapter le réglage de certains paramètres de fonctionnement depuis l'extérieur. A cet effet, la prothèse œsophagienne implantable comprend de préférence une interface agencée pour communiquer avec l'unité de commande de manière à établir des paramètres depuis l'extérieur qui peuvent différer de ceux qui seraient établis lors de l'initialisation de la prothèse.

Il peut également être envisagé de monter d'autres capteurs dans la prothèse. Ces capteurs seraient alors bien entendu reliés à l'unité de contrôle. Ainsi un accéléromètre peut être intégré pour détecter les déplacements du patient dans lequel la prothèse est implantée. Ainsi la gestion de la pression péristaltique appliquée peut tenir compte des déplacements du patient. Au lieu d'un accéléromètre, ou même en combinaison avec un accéléromètre on pourrait prévoir un gyroscope qui permettrait de détecter dans quel position, assis ou debout, le patient est. Le montage d'un capteur de température et/ou d'humidité peut également être envisagé.

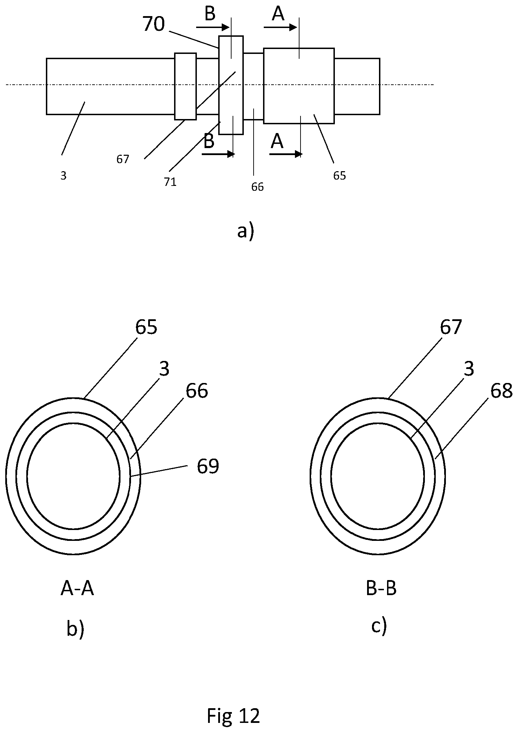

La figure 12a illustre une forme de réalisation particulière de la pompe 14. La figure 12b, respectivement 12c, montre une vue en coupe à travers la pompe selon la ligne A-A, respectivement B-B. Dans cette forme de réalisation la pompe est pour ainsi dire enroulée autour du tube alimentaire 3. La pompe comporte une entrée 70 qui sera reliée au réservoir de fluide 13 ainsi qu'une sortie 71 également reliée au réservoir de fluide. La pompe comporte un stator 65 qui fait partie d'un moteur électrique qui entraîne la pompe. Le stator est fixe et enroulé autour d'un rotor 66, comme illustré à la figure 12b. Le rotor est muni sur son pourtour intérieur d'au moins une protubérance 69 qui est en contact avec un anneau intérieur 68 qui fait partie de l'élément de pompage de la pompe. L'anneau intérieur étant enroulé d'un anneau extérieur 67 qui est fixe. Lorsque la pompe est activée le rotor 66 sera entraîné en rotation à l'intérieur du stator 65. La rotation du rotor va à son tour entrainer la protubérance 69 qui va ainsi exercer une action de pompage sur l'anneau intérieur 67 pour pomper le fluide. Comme la pompe est enroulée autour du tube alimentaire, le fluide ainsi pompé pourra directement être fournit à l'entrée de fluide 16. Le fait d'enrouler la pompe autour du tube alimentaire a comme avantage d'économiser de l'espace. Ceci est particulièrement intéressant dans le cas d'une prothèse d'œsophage implantable qui doit être implantée dans un corps humain où l'espace disponible est très limitée.

On observera qu'une telle forme de réalisation d'une pompe n'est pas limitée à une application dans une prothèse d'œsophage implantable et peut être appliqué à d'autres dispositifs comme des appareils électroménagers, des pompes médicales, et autres.

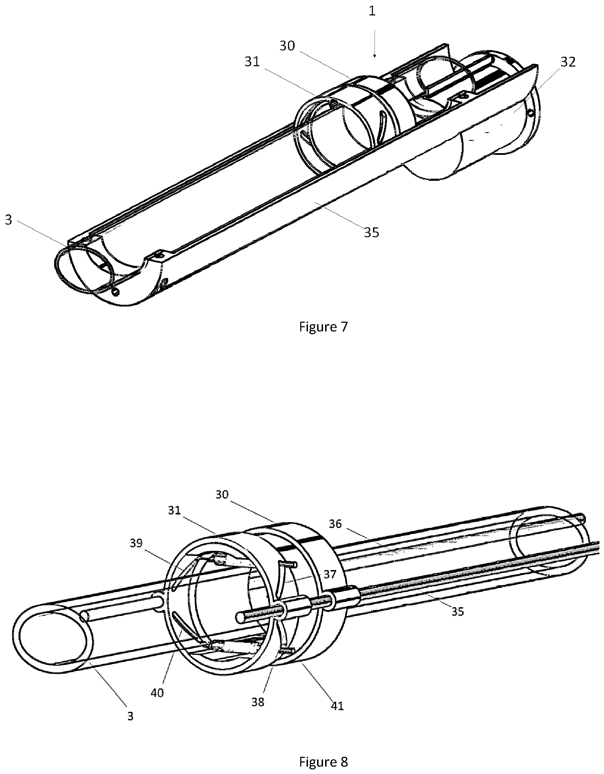

Selon un deuxième mode de réalisation préférentiel d'une prothèse d'œsophage implantable selon l'invention et représentée aux figures 7 à 10, l'élément d'application de pression comprend un poussoir 30 et un compresseur 31 montés sur un organe d'entraînement 32 agencé pour permettre le mouvement de haut en bas le long de l'aliment tube 3. Le compresseur 31 est monté en aval du poussoir 30 considéré dans la direction selon laquelle I1 s'étend. Le compresseur comprend un ensemble d'éléments de compression 33, 34 agencés pour exercer un mouvement d'aller-retour par rapport au tube alimentaire 3. Le poussoir étant agencé pour imposer un mouvement sur les éléments de compression afin de permettre ledit mouvement d'aller-retour.

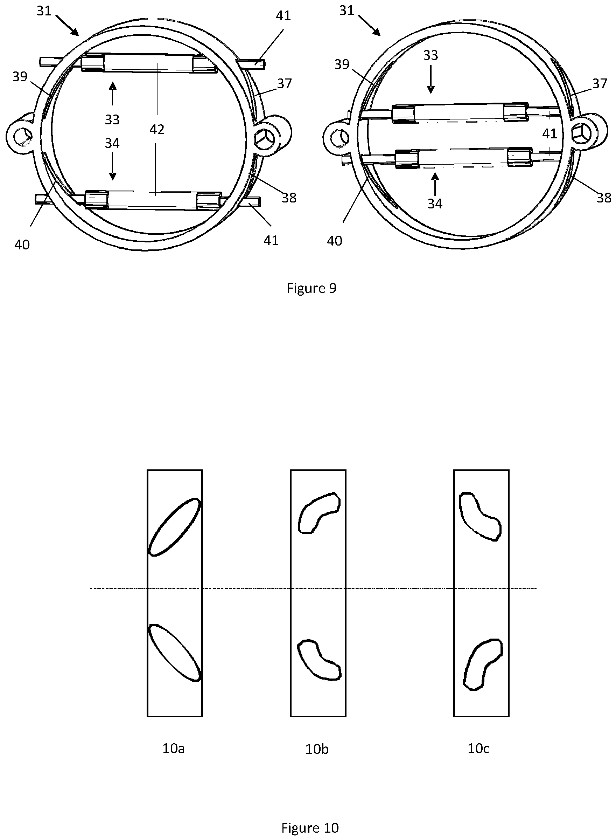

L'élément d'entraînement 32 comprend un moteur électrique connecté à une tige de compresseur 36 et une tige de poussée 35. La tige de compresseur 36 est connectée au compresseur 31 et la tige de poussée 35 est connectée au poussoir 30, comme illustré à la figure 8. Comme illustré aux figures 9a et 9b, le compresseur 31 comprend une bague munie d'un jeu de fentes 37, 38, 39 et 40 qui sont appliquées de manière à être inclinées par rapport à la première direction I1 dans laquelle se déplace l'élément d'application de pression. Les fentes 37 et 38, respectivement 39 et 40, sont appliquées de manière à former une forme en V par rapport à un axe médian de la bague. L'axe 41 de l'organe de compression 33, respectivement 34, est appliqué dans les fentes 37 et 39, respectivement 38 et 40 de manière à être déplacé à l'intérieur et guidé par les fentes dans lesquelles l'axe est appliqué. Les fentes 37 et 39, respectivement 38 et 40, sont appliquées de part et d'autre de la bague l'une par rapport à l'autre.

Sur la figure 7 et la figure 10a, les fentes s'étendent linéairement sur leur longueur. Il est cependant également possible que les fentes s'étendent selon une forme incurvée comme le montre la figure 10b, ou selon une forme concave comme le montre la figure 10c. La forme des fentes aura un effet sur le profil de couple appliqué par l'organe d'entraînement et donc sur la consommation d'énergie.

Au début du processus de compression, le compresseur 31 et le poussoir 30 sont séparés d'une distance prédéterminée, comme le montre la figure 7, ce qui permet aux organes de compression d'être placés sur l'extrémité supérieure, respectivement inférieure des fentes inclinées comme illustré sur la figure 9a. Dans cette position, aucune compression n'est appliquée sur le tube alimentaire. Lors d'une phase initiale de fonctionnement, le moteur électrique de l'organe d'entraînement 32 commence à faire tourner la tige de compresseur 36 en rotation au sens contraire des aiguilles de la montre, tandis que la tige de poussée 35 n'est pas entraînée. Ceci entraînera le déplacement du poussoir 30 vers le compresseur 31. Lors d'une première phase de fonctionnement, commençant une fois que le poussoir aura atteint le compresseur, la tige de poussée poussera sur l'axe sur lequel sont montés les organes de compression et les fera bouger à l'intérieur de la fente inclinée pour imposer un mouvement d'aller aux organes de compression.

En faisant cela, l'axe, sur lequel les éléments de compression sont montés, se déplace dans les fentes vers le centre du compresseur, comme illustré à la figure 9b, et comprime le tube alimentaire. Une fois la compression terminée, une deuxième phase opérationnelle est démarrée au cours de laquelle l'organe d'entraînement tourne dans le sens des aiguilles d'une montre et déplace à la fois le poussoir et le compresseur le long du tube alimentaire dans la direction I1, tout en maintenant le tube alimentaire sous compression pour ainsi réaliser la pression péristaltique continue contre la paroi du tube alimentaire. A l'issue de cette deuxième phase de fonctionnement, une troisième phase de fonctionnement est démarrée au cours de laquelle la tige de compresseur tourne dans le sens des aiguilles d'une montre tandis que la tige de poussée est à l'arrêt. L'effet est que le compresseur et le poussoir se séparent et que les organes de compression reviennent à leur position initiale dans les fentes. Par conséquent, le tube alimentaire est décompressé. Une fois la compression du tube alimentaire complètement relâchée, une quatrième phase opérationnelle est démarrée au cours de laquelle l'organe d'entraînement, qui joue alors le rôle d'organe de rappel, va imposer un mouvement au sens contraire des aiguilles de la montre au compresseur et au poussoir pour les ramener en position haute initiale. Les troisième et quatrième phases opérationnelles sont soit consécutives, soit coïncidentes dans le temps.

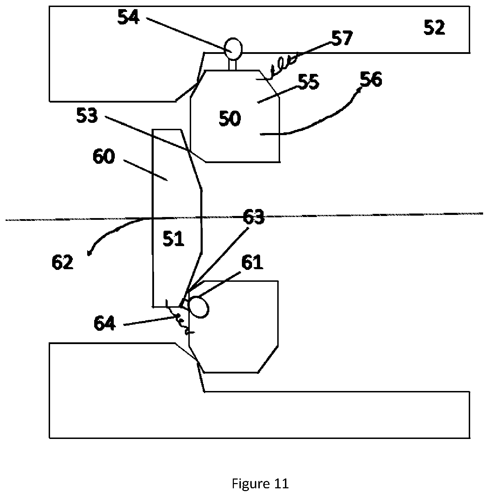

La figure 11 montre une coupe transversale à travers un exemple de la soupape de commande de passage d'aliments 7 à joindre au tube alimentaire en aval de la position finale 9 atteinte par l'élément d'application de pression lors de son déplacement le long du tube alimentaire. La soupape de commande de passage d'aliments 7 comprend un premier 50 et un deuxième 51 éléments de soupape. Le premier 50 et deuxième 51 éléments de soupape sont appliqués coaxialement l'un par rapport à l'autre. Le premier élément de soupape étant agencé pour permettre un premier passage du tube à l'estomac, tandis que le deuxième élément de soupape étant agencé pour permettre un deuxième passage de l'estomac au tube. De préférence, le premier et le deuxième élément de soupape s'étendent dans un même plan horizontal.

La figure 11 montre également que le premier élément de soupape 50 est logé dans un corps de soupape 52 pourvu d'un siège de soupape 53 formé par une cavité appliquée dans le corps de soupape. Le siège de soupape est de préférence de forme conique pour permettre une pression d'ouverture plus précise. Le premier élément de soupape comprend un premier élément d'articulation 54 appliqué de manière à pivoter dans le siège de soupape. Une première plaque de soupape 55 est reliée au premier élément d'articulation 54 et peut donc pivoter dans le tube alimentaire dans une direction vers l'estomac comme indiqué par la flèche 56. Un ressort de torsion 57 est appliqué entre la première plaque de soupape et le corps de soupape pour permettre un retour de la première plaque de soupape vers une position initiale où le premier élément de soupape est dans une position fermée. Le premier élément de soupape est de préférence calibré à une pression d'ouverture d'environ 25 mbar. La première plaque de soupape fermera complètement le tube alimentaire afin d'éviter un retour de l'estomac vers la bouche du patient.

Le deuxième élément de soupape 51 comprend une deuxième plaque de soupape 60 appliquée sur la première plaque de soupape 55. Le deuxième élément de soupape comprend un deuxième élément d'articulation 61 appliqué de manière à pivoter dans le premier élément de soupape. Une seconde plaque de soupape 60 peut pivoter dans le tube alimentaire dans une direction à partir de l'estomac, comme indiqué par la flèche 62. Le siège de soupape 63 dans lequel le deuxième élément d'articulation est logé est de préférence de forme conique. La pression d'ouverture de la seconde plaque de soupape est de préférence calibrée à une pression d'au moins 150 mbar, de préférence 200mbar. Un autre ressort de torsion 64 est appliqué entre la deuxième plaque de soupape et la première plaque de soupape pour permettre un retour de la deuxième plaque de soupape vers une position initiale où le deuxième élément de soupape est dans une position fermée.

On notera que la demande de brevet Français 2 621 813 décrit une soupape agencée pour être implanté dans une prothèse d'œsophage implantable. Toutefois la soupape connue ne connait pas une différence de pression d'ouverture qui dépend du sens de l'ouverture.

Il convient de noter que la soupape de commande de passage représentée sur la figure 11 ne convient pas seulement pour être utilisée dans une prothèse d'œsophage implantable et peut également être utilisée comme soupape de sécurité à usage général dans des conduits ou des dispositifs dans lesquels un fluide, un gaz ou même des matériaux solides sont circulé. La soupape de sécurité à usage général peut être utilisée dans des applications où un débit bidirectionnel doit être contrôlé, comme par exemple dans le pompage d'huile, le pompage de gaz, les flux de produits chimiques ou d'aliments. Bien entendu, la pression à laquelle la vanne s'ouvrira dépendra du domaine dans lequel la soupape est utilisée.

## Revendications

1. Prothèse (1) d'œsophage implantable comprenant un tube alimentaire (3), lequel tube alimentaire est au moins partiellement entouré d'un logement (2) dans lequel un organe pressuriseur (4,5) est appliqué, lequel organe pressuriseur comprend un élément d'application de pression (4) agencé pour générer une pression péristaltique contre une paroi externe du tube alimentaire, **caractérisée en ce que** l'organe pressuriseur est appliqué de telle manière à pouvoir être déplacé le long du tube alimentaire sur une distance prédéterminée à partir d'une position initiale (8), lequel élément d'application de pression (4) est agencé pour générer la pression péristaltique de façon continue pendant son déplacement le long du tube alimentaire dans une première direction (11) s'étendant vers l'estomac d'un corps dans lequel la prothèse doit être implantée, ledit organe pressuriseur comprenant un organe de rappel (5) agencé pour appliquer une force de rappel s'étendant dans une seconde direction ( I2), opposé à la première direction, pour ramener l'élément d'application de pression vers sa position initiale.

2. Prothèse d'œsophage implantable selon la revendication 1, **caractérisée en ce que** l'élément d'application de pression (4) est agencé pour générer la pression péristaltique continue en utilisant un fluide hydraulique ou un gaz sous pression.

3. Prothèse d'œsophage implantable selon la revendication 2, **caractérisée en ce que** l'élément d'application de pression (4) comprend une entrée de fluide (16) et une sortie de fluide (17), lequel élément d'application de pression comporte une paroi interne configurée pour envelopper au moins partiellement le tube alimentaire et pourvue d'au moins une première série de portes de sortie (24) et au moins une deuxième série de portes d'entrée (25), les portes d'entrée étant appliquées, considérées dans ladite première direction, sous les portes de sortie, les portes de sortie, respectivement d'entrée, étant reliée à ladite entrée de fluide (16), respectivement à ladite sortie de fluide (17), l'entrée de fluide étant reliée à une sortie d'une pompe (14) agencée pour fournir le fluide hydraulique à une pression d'au moins 2 bars.

4. Prothèse d'œsophage implantable selon la revendication 3, **caractérisée en ce que** la première série de portes de sortie (24) comprend au moins deux portes de sortie et la deuxième série de portes d'entrée (25) comprend au moins une porte d'entrée.

5. Prothèse d'œsophage implantable selon la revendication 3 ou 4, **caractérisée en ce qu'**elle comprend un capteur de pression (10) agencé pour mesurer une pression appliquée par des aliments présents dans le tube alimentaire, ladite prothèse comprend également une unité de contrôle (12) reliée audit capteur de pression, le capteur de pression étant agencé pour générer un premier signal de déclenchement sur base de la pression mesurée, ladite unité de contrôle étant agencée pour générer, sur la base dudit premier signal de déclenchement, un second signal de déclenchement ayant une amplitude et une période à fournir à la pompe (14) pour la gérer.

6. Prothèse d'œsophage implantable selon la revendication 3 ou 4, **caractérisée en ce que** la pompe (14) est enroulée autour du tube alimentaire (3).

7. Prothèse d'œsophage implantable selon l'une quelconque des revendications 1 à 6, **caractérisée en ce que** l'organe de rappel (5) est formé par un ressort logé à l'intérieur dudit logement.

8. Prothèse d'œsophage implantable selon la revendication 1, **caractérisée en ce que** l'élément d'application de pression comprend un ensemble d'organes de compression (33, 34) agencés pour exercer un mouvement d'aller-retour par rapport au tube alimentaire (3).

9. Prothèse d'œsophage implantable selon la revendication 8, **caractérisée en ce que** l'élément d'application de pression comprend un poussoir (30) et un compresseur (31) montés sur un organe d'entraînement (32) agencé pour générer ledit déplacement le long du tube, ledit compresseur étant monté en aval dudit poussoir, lesdits organes de compression faisant partie dudit compresseur, ledit poussoir étant agencé pour imposer le mouvement d'aller-retour auxdits organes de compression.

10. Prothèse d'œsophage implantable selon la revendication 9, **caractérisée en ce que** ledit organe d'entraînement est agencé pour pendant une première phase opérationnelle entraîner le poussoir pour imposer ledit mouvement d'aller sur lesdits organes de compression et pour pendant une seconde phase opérationnelle, à la suite de ladite première phase opérationnelle, imposer ledit mouvement le long du tube dans ladite première direction I1.

11. Prothèse d'œsophage implantable selon la revendication 10, **caractérisée en ce que** ledit organe d'entraînement est agencé pour lors d'une troisième phase opérationnelle, suite à ladite seconde phase opérationnelle, entraîner le poussoir pour imposer ledit mouvement de retour sur lesdits organes de compression et pour pendant une quatrième phase opérationnelle phase imposant ledit mouvement de rappel le long du tube alimentaire dans ladite deuxième direction I2.

12. Prothèse d'œsophage implantable selon la revendication 11, **caractérisée en ce que** lesdites troisième et quatrième phase opératoire sont soit consécutives l'une à l'autre, soit coïncidentes dans le temps l'une avec l'autre.

13. Prothèse d'œsophage implantable selon l'une quelconque des revendications 8 à 12, **caractérisée en ce que** les organes de compression sont formés chacun par un rouleau appliqué en rotation sur un axe (41).

14. Prothèse d'œsophage implantable selon la revendication 13, **caractérisée en ce que** le compresseur (31) comprend une bague munie d'un jeu de fentes (37, 38, 39, 40) qui sont appliquées de manière à être inclinées par rapport à la première direction, ledit ensemble de fentes comprend deux fentes par élément de compression appliquées sur les côtés opposés de ladite bague pour recevoir ledit axe.

15. Prothèse d'œsophage implantable selon l'une quelconque des revendications 1 à 14, **caractérisée en ce qu'**elle comprend une soupape de contrôle de passage alimentaire (7) placée dans ledit tube alimentaire en aval d'une position finale (9) atteinte par l'élément d'application de pression lors de son mouvement le long du tube alimentaire.

16. Prothèse d'œsophage implantable selon la revendication 15, **caractérisée en ce que** la soupape de contrôle du passage des aliments (7) comprend un premier et un deuxième élément de soupape, le premier élément de soupape étant appliqué coaxialement par rapport au deuxième élément de soupape, le premier l'élément de soupape étant agencé pour permettre un premier passage du tube alimentaire à l'estomac, le deuxième élément de soupape étant agencé pour permettre un second passage de l'estomac au tube alimentaire.

17. Prothèse d'œsophage implantable selon la revendication 16, **caractérisée en ce que** le premier, respectivement le deuxième élément de soupape est calibré à une pression d'ouverture d'environ 25 mbar, respectivement à une pression d'ouverture d'au moins 150 mbar, de préférence 200mbar.

## Patentansprüche

1. Implantierbare Speiseröhrenprothese (1) umfassend ein Ernährungsschlauch (3), welches Ernährungsschlauch zumindest teilweise von einem Gehäuse (2) umgeben ist in welches ein Druckorgan (4,5) angeordnet ist, welches Druckorgan ein Druckanwendungselement (4) enthalt angeordnet um einen peristaltischen Druck gegen eine Außenwand des Ernährungsschlauchs zu erzeugen, **dadurch gekennzeichnet, dass** das Druckorgan so angebracht ist um entlang des Ernährungsschlauchs über eine vorbestimmte Strecke aus einer Anfangsposition (8) bewegbar zu sein, welches Druckorgan (4) vorgesehen ist um kontinuierlichen den peristaltischen Druck zu erzeugen während seiner Bewegung entlang des Ernährungsschlauchs in einer ersten Richtung (11), die sich in Richtung des Magens eines Körpers erstreckt, in den die Prothese implantiert werden soll, welches Druckorgan ein Rückstellorgan (5) umfasst um eine Rückstellkraft in eine zweite Richtung (I2), die der ersten Richtung entgegengesetzt ist, auszuüben, um das Druckanwendungselement in seine Anfangsposition zurückzuführen.

2. Implantierbare Speiseröhrenprothese nach Anspruch 1, **dadurch gekennzeichnet, dass** das Druckanwendungselement (4) ausgelegt ist um den kontinuierlichen peristaltischen Druck unter Verwendung von hydraulischer Flüssigkeit oder Gas unter Druck zu erzeugen.

3. Implantierbare Speiseröhrenprothese nach Anspruch 2, **dadurch gekennzeichnet, dass** das Druckanwendungselement (4) einen Flüssigkeitseinlass (16) und einen Flüssigkeitsauslass (17) umfasst, wobei das Druckanwendungselement eine innere Wand aufweist, die so konfiguriert ist, dass sie den Ernährungsschlauch zumindest teilweise umschließt, und die mit mindestens einer ersten Reihe von Auslassöffnungen (24) und mindestens einer zweiten Reihe von Einlassöffnungen (25) versehen ist, welche Einlassöffnungen, in genannter ersten Richtung betrachtet, unterhalb der Auslassöffnungen angeordnet sind, welche Auslass-, bzw. Einlassöffnungen, mit dem genannten Flüssigkeitseinlass (16), bzw. dem Flüssigkeitsauslass (17) verbunden sind, wobei der Flüssigkeitseinlass mit einem Ausgang einer Pumpe (14) verbunden ist um sie die hydraulische Flüssigkeit mit einem Druck von mindestens 2 bar bereitzustellen.

4. Implantierbare Speiseröhrenprothese nach Anspruch 3, **dadurch gekennzeichnet, dass** die erste Reihe von Auslassöffnungen (24) mindestens zwei Auslassöffnungen und die zweite Reihe von Einlassöffnungen (25) mindestens eine Einlassöffnung umfasst.

5. Implantierbare Speiseröhrenprothese nach Anspruch 3 oder 4, **dadurch gekennzeichnet, dass** sie einen Drucksensor (10) umfasst ausgelegt um einen von in dem Ernährungsschlauch befindlichen Lebensmitteln ausgeübten Druck zu messen, welche genannte Prothese außerdem eine Steuereinheit (12) umfasst, die mit genanntem Drucksensor verbunden ist, welcher Drucksensor ausgelegt ist um auf Basis des gemessenen Drucks ein erstes Trigger Signal zu erzeugen, welche Steuereinheit ausgelegt ist um auf Basis des ersten Trigger Signals ein zweites Trigger Signal zu erzeugen, das eine Amplitude und eine Periode aufweist, die an die Pumpe (14) zur Steuerung ihrer Funktion zu liefern.

6. Implantierbare Speiseröhrenprothese nach Anspruch 3 oder 4, **dadurch gekennzeichnet, dass** die Pumpe (14) um den Ernährungsschlauch (3) gewickelt ist.

7. Implantierbare Speiseröhrenprothese nach einem der Ansprüche 1 bis 6, **dadurch gekennzeichnet, dass** das Rückstellorgan (5) durch eine Rückstellfeder gebildet ist, welcher innerhalb dem genannten Gehäuse untergebracht ist.

8. Implantierbare Speiseröhrenprothese nach Anspruch 1, **dadurch gekennzeichnet, dass** das Druckanwendungselement eine Anordnung von Kompressionsorganen (33, 34) umfasst angeordnet um eine Hin-und Her Bewegung relativ zum Ernährungsschlauch (3) auszuführen.

9. Implantierbare Speiseröhrenprothese nach Anspruch 8, **dadurch gekennzeichnet, dass** das Druckanwendungselement einen Schieber (30) und einen Kompressor (31) umfasst, die an einem Antriebsorgan (32) montiert sind und ausgelegt sind um die Bewegung entlang des Ernährungsschlauchs zu erzeugen, genannte Kompressor stromabwärts des Schiebers montiert ist, genannten Kompressionsorgane Teil des Kompressors sind, wobei genannter Schieber angeordnet ist um die Hin- und Her Bewegung auf die genannten Kompressionsorgane zu übertragen.

10. Implantierbare Speiseröhrenprothese nach Anspruch 9, **dadurch gekennzeichnet, dass** das genannte Antriebsorgan ausgelegt ist um während einer ersten Betriebsphase den Schieber anzutreiben, um die genannte Hin Bewegung auf die genannten Kompressionsorgane auf zu erlegen, und während einer zweiten Betriebsphase, die auf die erste Betriebsphase folgt, die Bewegung entlang des Ernährungsschlauchs in der genannten ersten Richtung (11) auf zu erlegen.

11. Implantierbare Speiseröhrenprothese nach Anspruch 10, **dadurch gekennzeichnet, dass** das genannte Antriebsorgan ausgelegt ist um während einer dritten Betriebsphase, die auf die zweite Betriebsphase folgt, den Schieber anzutreiben, um die Rückbewegung auf die genannten Kompressionsorgane zu übertragen, und während einer vierten Betriebsphase die Rückstellbewegung entlang des Ernährungsschlauchs in der genannten zweiten Richtung (I2) auf zu erlegen.

12. Implantierbare Speiseröhrenprothese nach Anspruch 11, **dadurch gekennzeichnet, dass** die genannten dritte und vierte Betriebsphase entweder aufeinanderfolgend oder gleichzeitig ablaufen.

13. Implantierbare Speiseröhrenprothese nach einem der Ansprüche 8 bis 12, **dadurch gekennzeichnet, dass** die Kompressionsorgane jeweils aus einer Rolle bestehen, die drehbar auf einer Achse (41) angebracht ist.

14. Implantierbare Speiseröhrenprothese nach Anspruch 13, **dadurch gekennzeichnet, dass** der Kompressor (31) einen Ring umfasst, der mit einer Anordnung von Schlitzen (37, 38, 39, 40) versehen ist, die dermaßen angebracht sind das sie geneigt zur ersten Richtung sind, welche genannte Anordnung von Schlitzen zwei Schlitze pro Kompressionselement umfasst, die an gegenüberliegenden Seiten des genannten Rings angebracht sind, um die genannte Achse aufzunehmen.

15. Implantierbare Speiseröhrenprothese nach einem der Ansprüche 1 bis 14, **dadurch gekennzeichnet, dass** sie ein Ventil zur Steuerung des Nahrungsdurchflusses (7) umfasst, das in dem Ernährungsschlauch stromabwärts von einer Endposition (9) angebracht ist, die das Druckanwendungselement bei seiner Bewegung entlang des Ernährungsschlauchs erreicht.

16. Implantierbare Speiseröhrenprothese nach Anspruch 15, **dadurch gekennzeichnet, dass** das Ventil zur Steuerung des Nahrungsdurchflusses (7) ein erstes und ein zweites Ventilelement umfasst, welches erste Ventilelement koaxial relativ zum zweiten Ventilelement angebracht ist, wobei das erste Ventilelement ausgelegt ist um einen ersten Durchfluss vom Ernährungsschlauch zum Magen zu ermöglichen, und das zweite Ventilelement ausgelegt ist um einen zweiten Durchfluss vom Magen zum Ernährungsschlauch zu ermöglichen.

17. Implantierbare Speiseröhrenprothese nach Anspruch 16, **dadurch gekennzeichnet, dass** das erste, beziehungsweise das zweite, Ventilelement kalibriert ist auf einen Öffnungsdruck von ungefähr 25mbar, beziehungsweise einen Öffnungsdruck von ungefähr 150mbar, vorzugsweise 200mbar.

## Claims

1. Implantable oesophageal prosthesis (1) comprising a feeding tube (3), which feeding tube is at least partially surrounded by a housing (2) in which a pressurising member (4,5) is applied, which pressurising member comprises a pressure application element (4) provided for generating a peristaltic pressure against an outer wall of the feeding tube, **characterised in that** the pressurising member is applied in such a manner as to be movable along the feeding tube over a predetermined distance starting from an initial position (8), which pressure application element (4) is provided for generating the peristaltic pressure continuously during its movement along the feeding tube in a first direction (11) extending towards the stomach of a body in which the prosthesis is to be implanted, said pressurising member comprises a return member (5) provided for applying a return force extending in a second direction (I2), opposite to the first direction, in order to return the pressure application element towards its initial position.

2. Implantable oesophageal prosthesis as claimed in claim 1, **characterised in that** the pressure application element (4) is provided for generating the continuous peristaltic pressure by using a hydraulic fluid or a gas under pressure.

3. Implantable oesophageal prosthesis as claimed in claim 2, **characterised in that** the pressure application element (4) comprises a fluid input (16) and a fluid output (17), which the pressure application element comprises an internal wall configured for at least partially enveloping the feeding tube and provided with a first series of output gates (24) and at least a second series of input gates (25), the input gates being applied, considered in said first direction, under the output gates, the output gates, respectively the input gates, being connected to said fluid input (16), respectively to said fluid output (17), the fluid input being connected to an pump output (14) provided for furnishing the hydraulic fluid at a pressure of at least 2 bars.

4. Implantable oesophageal prosthesis as claimed in claim 3, **characterised in that** the first series of output gates (24) comprises at least two output gates and the second series of input gates (25) comprises at least one input gate.

5. Implantable oesophageal prosthesis as claimed in claim 3 or 4, **characterised in that** it comprises a pressure sensor (10) provided for measuring a pressure applied by food present in the feeding tube, said prosthesis also comprises a control unit (12) connected to said pressure sensor, the pressure sensor being provided for generating a first trigger signal on the basis of the measured pressure, said control unit being provided for generating, on the basis of said first trigger signal, a second trigger signal having an amplitude and a period to be furnished to the pump (14) for controlling the latter.

6. Implantable oesophageal prosthesis as claimed in claim 3 or 4, **characterised in that** the pump (14) is wrapped around the feeding tube (3).

7. Implantable oesophageal prosthesis as claimed in anyone of the claims 1 to 6, **characterised in that** the return member (5) is formed by a spring lodged at the inside of said housing.

8. Implantable oesophageal prosthesis as claimed in claim 1, **characterised in that** the pressure application element comprises a set of compression members (33,34) provided for exerting a back and forth movement with respect to the feeding tube (3).

9. Implantable oesophageal prosthesis as claimed in claim 8, **characterised in that** the pressure application element comprises a pusher (30) and a compressor (31) mounted on a driving member (32) provided for generating said movement along the tube, said compressor being mounted downstream of said pusher, said pressure members being part of said compressor, said pusher being provided for imposing the back and forth movement to said compression members.

10. Implantable oesophageal prosthesis as claimed in claim 9, **characterised in that** said driving member is provided for driving the pusher during a first operational phase for imposing said forth movement on said compression members and for, during a second operational phase, following the first operational phase, imposing said movement along the tube in said first direction I1.

11. Implantable oesophageal prosthesis as claimed in claim 10, **characterised in that** said driving member is provided for during a third operational phase, following said second operational phase, driving the pusher for imposing said back movement on said compression members and for during a fourth operational phase imposing said return movement along the feeding tube along said second direction I2.

12. Implantable oesophageal prosthesis as claimed in claim 11, **characterised in that** said third and fourth operational phases are either subsequent to each other, or coincident in time with each other

13. Implantable oesophageal prosthesis as claimed in anyone of the claims 8 to 12, **characterised in that** the compression members are each formed by a roll applied in rotation on an axis (41).

14. Implantable oesophageal prosthesis as claimed in claim 13, **characterised in that** the compressor (31) comprises a ring provided with a set of splits (37,38,39,40) which are applied in such a manner as to be inclined with respect to the first direction, said set of splits comprising two splits per compression element applied on opposite sides of said ring for receiving said axis.

15. Implantable oesophageal prosthesis as claimed in anyone of the claims 1 to 14, **characterised in that** it comprises a food passage control valve (7) applied in said feeding tube downwards a final position (9) reached by the pressure application element during its movement along the feeding tube.

16. Implantable oesophageal prosthesis as claimed in claim 15, **characterised in that** the food passage control valve (7) comprises a first and a second valve element, the first valve element being coaxially applied with respect to the second valve element, the first valve element being provided for enabling a first passage of the feeding tube to the stomach, the second valve element being provided for enabling a second passage from the stomach to the feeding tube.

17. Implantable oesophageal prosthesis as claimed in claim 16, **characterised in that** the first, respectively the second valve element is calibrated at an opening pressure of about 25mbar, respectively an opening pressure of at least 150mbar, preferably 200mbar.
